# EUROPEAN PATENT SPECIFICATION

(11) **EP 3 397 339 B1**
(45) Date of publication and mention of the grant of the patent: **12.07.2023**
(21) Application number: 16849908.5
(22) Date of filing: 30.12.2016
(51) Int. Cl.: A61N 1/32, A61B 90/00, A61B 5/00, A61N 1/05, A61N 1/04, A61B 5/01, A61B 5/0537, A61B 5/145

(54) **THERANOSTIC ELECTROPORATOR WITH BIPHASIC WAVE FOR DIAGNOSIS OF PATHOLOGICAL AREAS AND TRANSFER OF THERAPEUTIC MOLECULES**
THERANOSTISCHER ELEKTROPORATOR MIT BIPHASISCHER WELLE ZUR DIAGNOSE PATHOLOGISCHER BEREICHE UND TRANSFER THERAPEUTISCHER MOLEKÜLE
DISPOSITIF D'ÉLECTROPORATION THÉRANOSTIQUE À ONDE BIPHASIQUE POUR LE DIAGNOSTIC DE ZONES PATHOLOGIQUES ET LE TRANSFERT DE MOLÉCULES THÉRAPEUTIQUES

(30) Priority: 31.12.2015 IT UB20159819
(43) Date of publication of application: 07.11.2018
(73) Proprietor: Biopulse S.r.l., 00144 Roma (RM) (IT)
(72) Inventor: SPUGNINI, Enrico, Pierluigi, 80132 Napoli (IT); BALDI, Alfonso, 80132 Napoli (IT)
(74) Representative: De Tullio, Michele Elio
(86) International application number: PCT/IB2016/001822
(87) International publication number: WO 2017/115124

(56) References cited:
- WO-A1-2010/097140
- US-A1- 2008 091 135
- US-A1- 2009 149 797
- US-A1- 2014 277 219
- US-B1- 6 949 081

## Description

### Field of application

The present invention belongs to the technical field of electrochemotherapy and, particurarly, electrochemotherapy performed by electroporation.

More particularly, the present invention relates to a theranostic device to be used for therapeutic treatments purposes, carried out by the transfer of therapeutic molecules by means of electroporation.

### Technical field

Electrochemotherapy (ECT) is a kind of chemotherapy which allows the local therapeutic transfer (drug delivery) of molecules and, in particular, of non-permeating medicines such as anticancer, anti-inflammatory agents, hormones, cytokines, immunoregulatory, antibodies, DNA, etc., inside a cell by means of electroporation. The electroporation is based on the local application of electrical modulated pulses, having high voltage and low amperage, capable of temporarily alter the cell membrane and promote the formation of the so-called "aqueous channels", also known as "electro-pores", allowing the selected substances (water-soluble molecules of active substances) to be carried into depth, based on the problem to be solved, not only of neoplastic nature, but also of an algic origin, inflammatory, etc.

In other words, when a biological cell is exposed to an electric field of sufficient intensity, it generates an increase of its transmembrane voltage, which brings the structure of the membrane to an increase of its permeability, not allowing not-permeant molecules to enter into the cell; it is possible to optimize the electroporation parameters for the different isotypes, by varying the intensity of the electric field and the duration of treatment.

Electroporation or elettropermeabilization has become broadly used in order to improve the administration of anti-cancer drugs into cells by means of a specific instrument, called electroporator.

A medical electroporator is based on the use of electrodes which, once introduced in the pathological tissue that is formed after the proliferation of primary tumors, create electric fields that "open" the pores in the cell membrane and thus allow an easier and increased drugs injection into the diseased cells, thus enhancing the efficacy.

Currently, the most investigated drug in electrochemotherapy is bleomycin.

This molecule is, from a chemical point of view, lipophobic, and enters into the neoplastic cells through a membrane carrier inducing a single and / or double breakage of the double helix of DNA, which leads to death of the target cell.

When bleomycin penetrates (in limited quantities) in tumor cells, due to carrier protein, the cell stops in the G2-M phase of the cell cycle, expands and often becomes multinucleate before dying, as happens to lethally irradiated cells.

When, following electroporation, million of bleomycin molecules enter in the cell, the cell dies by apoptosis.

The factors that identify the electroporation are length, number and waveform, wherein the first two act on the permeabilization threeshold, while the role of the waveform is still not yet completely clarified.

At the cellular level, the impulse initially generates transient electropores which, once stabilized, provide long-lasting electropores.

The second one, of greater duration, allows the passage of small or intermediate molecules; the generally employed waveform for this purpose is the square wave, which allows a pulse with better uptake as regards the non-homogeneous alignment of tumor cells compared to the alignment of the electric field.

In general, it can be said that the efficiency is related to the shape and the amplitude of permeabilizing pulse, as well as voltage and the coefficient of the membrane poration.

From the electrodes point of view, it has been pointed out that the plate electrodes (or caliber) allow a more homogeneous electric field than the needle electrodes, this allowing a greater permeabilization efficiency.

Some studies have been carried on on humans for the treatment / palliation of skin cancers including metastatic melanoma, basal cell carcinoma, head carcinomas and neck carcinomas of the salivary glands and breast using electrochemotherapy with square wave generators.

### State of the Art

US patent application No. 2009/149797 entitled "Method and system for modulating neural activity" discloses systems and methods for modulating neural activity by repetitively blocking conduction in peripheral neural structures with chemical blocking agents. It discloses in addition systems and methods for reversing effects of chemical blocking agents and/or for producing substantially permanent conduction block. Said patent application doesn't disclose the use of sensors for detection aimed at diagnostic and prognosis purposes of pathologies or, more generally, aimed at detecting the characteristics of the lesion and the area to be treated, in order to define a specific therapeutic protocol, taking into account the specific features of the individual case.

U.S. patent application No. 6241701 entitled "Apparatus for electroporation mediated delivery of drugs and genes', discloses a method and an apparatus for therapy in a living organism by electroporation.

In one embodiment of the apparatus according to US6241701, it is described a method for using the EPT (therapy by electroporation) with low voltage and long duration pulses to determine the cell death; in particular, a system for clinical electroporation that includes an electrode provided with a set of needles and having a "keying" element that determines the set point of the pulse voltage according to therapy is disclosed.

International Patent Application No. 2010118387, entitled "Integration of very short electric pulses for minimally to noninvasive electroporation", describes methods, devices and systems for the in vivo treatment of proliferative dysfunction cells and for the treatment of cancer; those methods including the integration of ultrashort electrical pulses, both temporally and spatially, to obtain the desired mode of cell death.

However, themethods above, are used for the treatment of tumors by means oftherapies which provide the application of non-thermal irreversible electroporation (IRE).

The Electroporation through biphasic pulse train is known from patent lieterature No. EP2221086, entitled "Portable equipment comprendendo generator circuit and provider for transfer of therapeutic molecules by electroporation", disclosing an handheld apparatus for gene electrochemotherapy in animals through the local therapeutic transfer of drugs by means of electroporation.

This equipment generates permeabilasing trains of electrical pulses inducing transient perturbations of the cytoplasmic membrane of tumor cells, thus increasing the absorption of anticancer molecules.

The pulses are provided by a circuit that includes a rechargeable battery that powers a capacitor to generate a train of biphasic pulses to high voltage electrodes, which require only one control on the voltage of the generated pulses.

On the other hand, International patent application No. 2009005568 shows methods and apparatuses for the ablation of tissues, primarily in order to increase the permeability of the surface barrier.

The embodiments illustrated in the aforesaid document, allow the rapid thermomechanical ablation of a tissue by a microfluidic jet generated by an arc discharge to generate microscopic holes - in the order of microns - localized on the level of the horny layer, thus increasing the permeability of the fabric itself.

The non-patent literature document entitled "Electrochemotherapy of skin tumors: comparison of two electroporation protocols" from "Official Journal of the Balkan Union of Oncology" - [2004, 9 (1): 47-50] - illustrates a study related the effects of a sequence of electrical pulses for the purpose of treatment of skin tumors in non-primary melanoma, using electrochemotherapy.

According to said study, the electrochemotherapy efficiency in non-melanoma skin cancer depends on the duration of the electrical pulses.

Ultimately, the majority of devices currently used in clinics, generate individual pulses: this causes a significant increase in the duration of the therapeutic sessions (and, consequently, of the anesthesia administered to the patient), and, also, an increased morbidity of the patients.

A factor that resulted as limiting factor mainly resides in the pain caused by the impulse (with the consequent need of sedation) and the electrical stimulation of muscle fibers / nerve centers, underlying neoplastic lesion.

It should also be pointed out that the above-mentioned electro tools use disposable electrodes which, among the rest, are not suitable for intra-operative use.

Moreover, the treatments carried out by the above-mentioned equipment are extremely painful for the patient, who is exposed to painful muscular electrostimulation (with consequent contracture), due to the use of monophasic pulses instead of biphasic pulses.

All the devices and the methods described in the patent documents above, and further ones that present in the state of the art, are also limited to the mere application of therapeutic treatments and elettrochemotherapic methods, such as electroporation for the treatment of pathological conditions.

Certain diseases, such as cancer, are however extremely heterogeneous, and existing treatments result effective only for a limited number of patients in relation to the different states of the disease. Therefore, tumors are treated with multi-modeless approach in which the surgery is associated to radiotherapy (brachytherapy or radiation therapy).

Chemotherapy is used as an adjuvant in neoplasia with a tendency to metastasize or as a neoadjuvant, to increase the chances of eradication.

Another critical issue in assessing the local control problem in cancer patients is the biological cost (and often also psychological) necessary to reach the predetermined clinical outcome.

Often, patients must undergo disfiguring or, even, mutilativing surgery (eg. amputation of limb); also, the complications associated with radiotherapy may be the cause of significant morbidity.

The most common side effects that patients undergoing radiotherapy suffer are: local fibrosis, necrosis, neuropathy radiation-induced and stomatitis radiation-induced; rarely radiation-induced tumors have been described.

In the current state, the diagnosis and monitoring of tumor lesions are gaing a great medical interest, further thantheir treatment by means of the above mentioned methods and, in particular, several studies offered an opportunity to approach the diagnosis to treatment through a new discipline known with the term "theranostics" (therapy and diagnostics).

With the theranostics the possibility there is of establishing a very close bond between diagnosis and therapy in order to obtain more specific therapeutic protocols for individual patients who capable to offer a higher probability of a better prognosis. Studies in the field of theranostics allowed the developing of specific customized therapies that allow to early identify cancer cells by the use of magnetic nanoparticles.

The magnetic particles are used as contrast agents in nuclear magnetic resonance to enhance the image contrast and thereby permitting a better detection of the tumor.

It is expected that the development of this technology will allow the identification of the tumor during the first stages of its development, or when it is still spread at a level of a few cells.

At the same time, the magnetic nanoparticles can also be used to spread the therapy, for example by applying the magnetic hyperthermia, a technique that is based on warming, by means of the application of a weak alternating magnetic field of a suitable frequency.

The nanoparticles can be properly functionalized with a polymer coating with the aim of delivering drugs in a targeted manner, to be released when necessary.

As an example, some patent documents of the prior art that disclosing methods and devices for electroporation, configured to run both the local treatment or for the detection of physical-chemical and physiological parameters are listed below.

U.S. Patent No. 9198733B2 discloses a configuration includinf planning systems for the treatment and devices for the determination of a patient-specific treatment protocol comprising:
a) a module, configured to receive and process information from medical images of a target structure, to prepare a reconstruction of a 3-D model of the target structure;
b) a module, operatively configured forperforming an analysis of the numerical model, by meanso of using, as inputs in analyzing the 3-D reconstruction and information from one or more of the physical constraints, tissue heterogeneity, dynamic effects of electrical permeabilitation, dynamic thermal effects or effects resulting by multiple treatments;
c) a module, operatively configured to build one or more electrical protocols which define a treatment region, and the respective treatment parameters to effectively treat the target structure.

U.S. Patent No. 9186213B2 discloses a method for the treatment of hypertension in a human patient which includes the placement of a device capable of providing an electric energy field aiminig at reducing the patient's blood pressure.

The method discloses the monitoring of parameters such as power, temperature, impedance and, if necessary, the modification of the energy field that is applied based on the values of the above parameters.

US patent application No. 20150088120A1 discloses systems and methods for the modeling and graphic representation of the tissues' heating and of the electric field distribution in treatment devices which apply electrical energy through one or a plurality of electrodes.

The methods includes: providing one or more parameters of a treatment protocol for the provision of one or more electrical pulses to the tissue through a plurality of electrodes; shaping the distribution of electricity and heat in the tissue on the basis of the parameters; providing a graphical representation of the electrical distribution and the modeled heat.

In a configuration of the system according to US20150088120A1, a planning module of treatment is configured to generate an ablation target area, determined on the basis of a combination of one or more parameters of a irreversible electroporation protocol and one or more specific tissue - conductivity parameters.

### Summary of the invention

The invention is defined in the appended claims. Aspects, embodiments and examples disclosed herein which do not fall within the scope of the appended claims do not form part of the invention, and are merely provided for illustrative purposes.

In the view of the above, an object of the present invention is to provide a theranostic apparatus that allows the combination of diagnostic, therapeutic and monitoring phases of different pathologies, optimizing the effectiveness of the therapeutic treatment to be performed and the related customization, in particular on the basis of the local features of the area to be treated.

The above-mentioned objective is achieved by means of a theranostic apparatus for the therapeutic transfer of molecules by meanso of electroporation, having the features of claim 1.

In one embodiment of the present invention, said at least one treatment probe is provided with an electrode comprising two or four needles made of medical-grade steel, partially covered with electrically insulating ceramic glass, the ends of said needles being made of or covered with an electrically conductive material. Alternatively, said at least one treatment probe is provided with an electrode comprising two plates of partially medical-grade steel covered with electrically insulating ceramic glass, the ends of said plates being made of or covered with an electrically conductive material.

In accordance with the present invention, said at least one treatment probe is configured to deliver electrical pulses sequences with an energy release regulated according to an incremental mode and, preferentially, it is configured for the execution of at least three amperage value measurements in the first 50 µs of the electroporation pulse sequence.

According to the present embodiment, said microcontroller unit is configured to provide the automatic interruption of the electroporation pulses delivery an amperage value equal to or greater than five ampere is overcome.

According to the invention, said one or more sensors can be configured for measuring, individually or in combination, one or more parameters relating to temperature, pH, impedance, oxygen concentration, vascularization, respectively for the determination of the phenotyping of pathologies.

Said one or more sensors can be invasive, configured for measuring physiological parameters by means of insertion, respectively non-invasive, configured for measuring physiological parameters through transcutaneously.

Preferably, said apparatus is configured for the automatic recognition of the type of probes and sensors connected to the same and for the acquisition of the relevant operational information.

In addition, said at least one treatment probe, respectively said at least one measurement probe, can comprise a respective RFID module for tracking and storing the number of uses in a predetermined time unit.

Advantageously, the microcontroller unit is configured for the determination and control of an electroporation pulses sequence for the loco-regional treatment of disorders comprised in the group of solid neoplasms, visceral and pararectal tumors, benign or low malignancy solid viral growths, benign or low malignancy lesions. According to the present embodiment, said storage means of said main body, operatively connected to said microcontroller unit, are configured to include a database for the storage of a plurality of physiological parameters, detected by said sensors, before, respectively after, the delivery of the electroporation pulses sequence, upon the instructions from the microcontroller unit. Said microcontroller unit is implemented with software means configured for the processing of said parameters and for the subsequent automatic adjustment of the electroporation pulses sequence, delivered on the basis of the same.

Said probes may include codification means, configured to allow said apparatus to recognise the type and the number of sensors included.

In a preferred configuration of the present embodiment, said apparatus further comprises a clock/calendar unit, configured for providing said microcontroller unit with a plurality of temporal location information related to a plurality of parameters, stored in said storage means, a power control unit, able to convert signals generated by said microcontroller unit into corresponding power pulses, a current sensor unit, configured for the detection of at least one value related to the amount of supplied current and for the transmission of at least said one value to said microcontroller unit, said microcontroller unit being configured for the reception and for the intelligence of said at least one value, and for the subsequent limitation of the amount of supplied energy, when said at least one value exceeds a predetermined threshold.

### Brief description of the drawings

Further characteristics and advantages of the invention will become more evident on the basis of the detailed description of a preferred embodiment, tof an apparatus for the diagnosis of tumoral areas and the therapeutic transfer of molecules by means of electroporation, illustrated as a non-limiting example with reference to the attached drawings wherein:

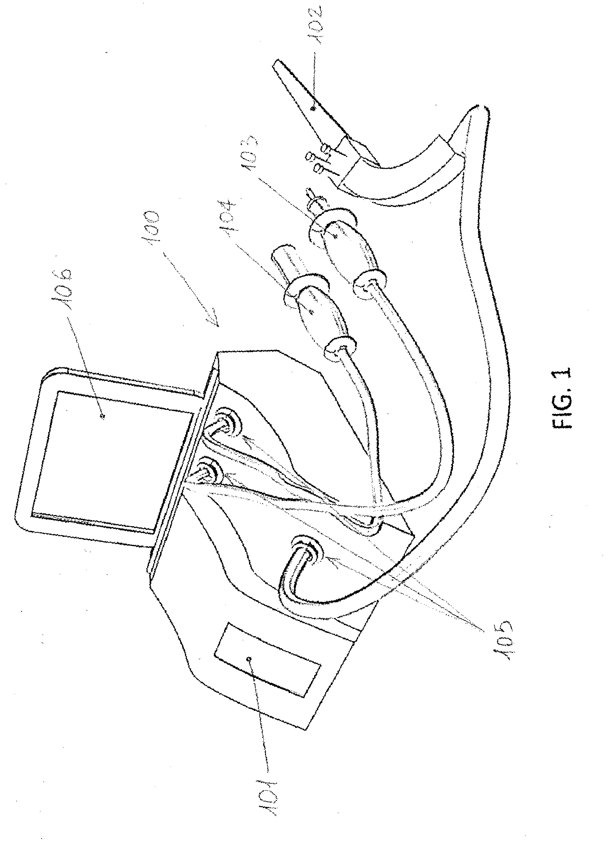

FIG. 1 is an axonometric view of an apparatus for the diagnosis of tumoral areas and the therapeutic transfer of molecules by means of electroporation according to one embodiment of the present invention;

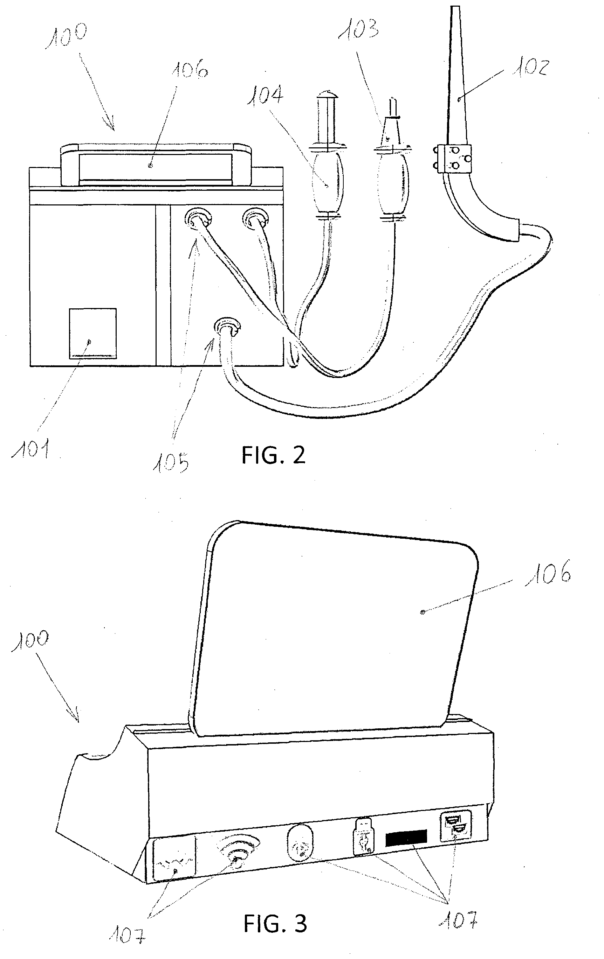

The FIG. 2 and 3 are respectively a top and a rear view of the apparatus of figure 1;
FIG. 4 is a schematic view of the hardware-software components of the apparatus and respective operating connections;
FIG. 5 is a simplified schematic view of the hardware-software components of the apparatus and respective operating connections

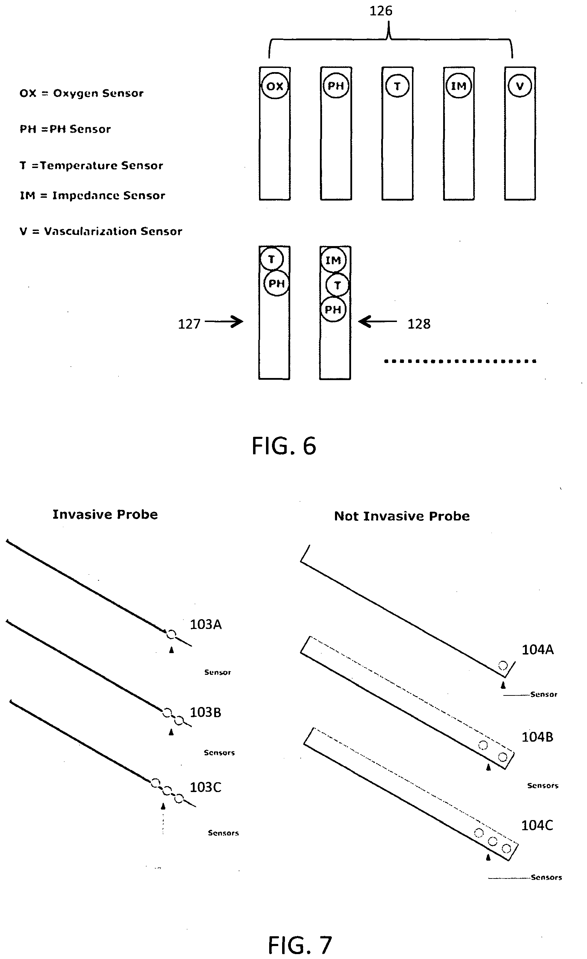

The FIG. 6 and 7 are schematic views of the configurations of the sensors of the apparatus;
FIG. 8 is a simplified schematic view of the operation of the firmware and its operational connecrtions with hardware and software means;
FIG. 9 is a flow diagram relating to an illustrative implementation of a method for the therapeutic transfer of molecules by electroporation, according to the present invention.

### Detailed description of a preferred example of embodiment

With reference to the cited drawings it is provided, in the following, a description of an exemplary embodiment of the apparatus for the diagnosis of pathological areas and the therapeutic transfer of molecules by electroporation, according to the present invention.

Said apparatus comprises, as know from the prior art:
- a main body 100, provided with electronic processor means and data storage means,
- input means 101, in the form of keyboard and/or touch screen,
- at least one treatment probe 102, provided with an electrode, said electrode delivering electrical pulses sequences by means of electroporation,
- a plurality of probe connectors 105, for said at least one treatment probe 102,
- hardware and software means 106, configured for displaying data, respectively providing sound signals,
- connection means 107 with respect to an internet network for data transmission using the same,
- a power supply circuit, including batteries and interface for connection to an external power source, and
- a micro-controller unit 108, operationally connected to said electronic processor means and said data storage means.

As an example, said hardware resources for data visualization, respectively for the generation of sound signals, may comprise an intelligent mobile device, such as a tablet computer or a smartphone, respectively different resources to electronic processor, known to the state of the art. Said microcontroller unit is advantageously implemented with a firmware, suitable to be updated remotely in an easy way for the operator, for example using a personal computer or something similar.

According to the present invention, said apparatus comprises at least one detection probe 103, 104, said probe comprising one or more sensors 125, 126, configured for measuring a plurality of physiological parameters having prognostic value in a subject undergoing the treatment. Conveniently, said microcontroller unit 108 is configured for the acquisition and processing of said physiological parameters having prognostic value, measured by said at least one detecting probe 103, 104, and for the subsequent generation and control of electroporation pulses sequence, on the basis of said processed physiological parameters having prognostic value for the loco-regional treatment of disorders comprised in the group of solid neoplasm, visceral and pararectal tumors, benign or low malignancy solid viral growths, benign or low malignancy lesions.

.Advantageously, the probe, provided with an electrode 102, may assume two configurations: plate-shaped or needle-shaped.

In particular, the plate electrodes comprise an anatomical hand-grip on which two medical grade steel plates, covered glass ceramic having a functional end including a rectangular surface that is not coated with electrically insulating material, engage. The electric conduction takes place through this metal surface.

On the other hand, the needle electrodes may be configured according to a configuration comprising two or four needles having a preferred length equal to 10 cm, conveniently separated by a distance inter-needle of 1.5 cm and, moreover, coated with glass ceramic. The end of each needle is uncovered and represents the functional part.

The configuration with two needles is particularly useful for the treatment of ecoassisted visceral lesions.

More specifically, said configuration is highly effective for the treatment of mediastinum lesions without inducing cardiac arrhythmia following the confinement of the passage of power in the area between the two needles.

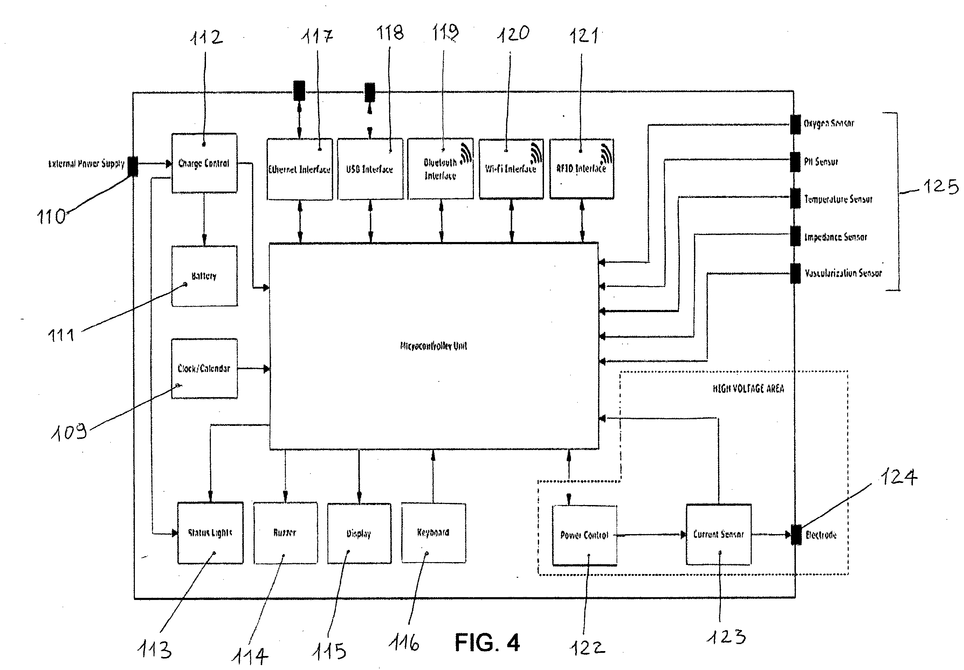

According to a preferred configuration of the present invention, as schematically represented in figure 4, said apparatus further comprises:
- A clock/calendar unit 109, configured for providing said microcontroller unit 108 with a plurality of temporal location information relating to a plurality of parameters stored in said storage means;
- External power supply means 110 (charger), configured for controlling the charging of the battery 111 through a charge control circuit 112, which also informs the user about the state of charge of the battery, by means of luminous user;
- Integrated keyboard means 116, configured for the inputting information from the user's side;
- Means for receiving and transmitting data, including: ethernet interface 117 for connecting to a LAN, USB interface 118 for connection with PC and a pendrive, Bluetooth interface 119 for connection with PC and/or mobile phones, Wi-Fi interface 120 for connection with PC and mobile phones, RFID module 121 for reading and writing data on card equipped with a transponder;
- A power control unit 122, adapted to convert the signals generated by said microcontroller unit 108 in corresponding power pulses;
- A current sensor unit 123, configured for the detection of at least a relative value of the amount of supplied current and for the transmission of said at least one value to said microcontroller unit 108, said microcontroller unit 108 being configured for the reception and the intelligence of said at least one value and for the consequent limitation of the amount of supplied energy, in the case said at least one value exceeds a predetermined threshold. Conveniently, the apparatus further comprises suitable means to provide the user with information relating to possible faults due to the excessive power delivery or lack of power delivery.

The electrode 124 is configured to provide the pulses by means of electroporation, said pulses being generated by the microcontroller unit 108 and being characterised by parameters that can be set by the user, which control amplitude, the number and duration of the pulses.

The detection sensors 125 connected to the apparatus, are advantageously configured for the measurement of temperature, pH, impedance, concentration of oxygen, vascularization and/or other parameters useful for diagnosis, prognosis and monitoring of pathologies.

The microcontroller unit 108 is suitable for implementing the necessary functions for the operation of the apparatus, such as:
- Acquisition of measurement data coming from the sensors,
- Generation and control of the electroporation pulses,
- Checking the charge state of the battery,
- Processing and local data storage,
- Communication with the user via input/output devices,
- External interfacing via Ethernet connections, USB, Bluetooth, Wi-Fi and RFID.

The charge control circuit 112 is advantageously capable to provide information to the microcontroller unit 108 regarding the voltage value of the battery 111 and the charge level of the same.

In the case in which the battery 111 is charging, the microcontroller unit 108 inhibits all the functionality of the apparatus so as to prevent its use until a connection condition with a power supply network, by means of a charger 110 remains.

The microcontroller unit 108 is capable to automatically recognize the type of probe connected 102, 103, 104 and the corresponding sensor 125, 126, and acquire the information for the operation.

Said information is used to define the treatment according to the specificity of local conditions, giving indications to the operator regarding the more suitable electroporation settings in the light of the charachteristics of the specific case.

Said sensors are housed inside the probes 103, 104, which may have different shape and be made with different types of material.

The RFID module 121 allows the traceability of consumables in order to avoid multiuse; in this way, once the consumable (in particular, the different types of electrode needles, such as the gun with "caliper" electrode) is activated by means of the intelligence of an appropriate code using the RFID module, so that the device begins to store the use of consumables (counting) and allows a limited use in accordance with the current setting values.

The consumable has a predefined duration in order to maximize the application efficiency in man, as well as to ensure the sterility that derives from the characteritics of the consumable to be disposable. In the veterinary field it, r the so-called "sharpness" has also to be taken into consideration.

The apparatus is suitable to operate according to the "just about system mode", releasing incrementally sufficient energy to overcome the resistance of the treated fabric, thus ensuring the passage of energy without causing durable side effects.

For this purpose, the apparatus is provided with a firmware means which measure the amount of released energy, by performing three measurements in the first 50 µs of the sequence of pulses. If the amperage value exceeds five amperes, the unit will shut down automatically the release of subsequent electrical pulses, informing the operator with sound signal and an "overcurrent" message alert on the display.

In clinical practice, it has been noted that this feature is activated during the treatment of necrotic areas or highly sclerosed, potentially proclaimers of resistance to treatment.

The operator will therefoere intervene by changing the electrical parameters and the orientation of the electrodes, respectively the type of the same (for example, needle or plate) to overcome this resistance.

In a further configuration of the present embodiment it is possible to connect the detection probes equipment that comprise a sensor 126 and / or two sensors 127 and / or more sensors 128 in order to simultaneously acquire a higher number of measurements, and thus simplify and speed up the measuring operations.

Also in this case the equipment will be capable to automatically recognize the type of the connected probes and the corresponding sensors.

Particularly, as a function of the operating conditions, it becomes necessary to provide two types of detection probes:
- Probes equipped with one or more invasive sensors - 103A, 103B, 103C - wherein said sensor is made to penetrate inside the part for which the acquiring of a measurement is needed. The detection of the parameters is made by means of insertion terminal.
- Probes equipped with one or more sensors of a not invasive type - 104A, 104B, 104C, in which said sensor is applied on the part to be measured without penetrating. The detection takes place transcutaneously through a contact terminal area, placed on the skin.

The detection probes that operate by means of insertion will allow evaluating the various physico-chemical parameters in the different areas of the lesions (eg. periphery versus center).

The data collected with the detection probes have diagnostic and prognostic value, and allow to accordingly choose the electroporative optimized protocols.

Each probe 103, 104 is equipped with a coding mechanism that enables the equipment to recognize the type and the number of sensors contained in it.

In a preferred embodiment, the treatment probe that is used is equipped with an electrode for therapy having a glass ceramic coating, which replaces the normal polymer coating.

Said glass ceramic electrode allows to use of the device below 800 (Volt / cm).

The glass ceramic electrode has an excellent electrical shielding, and has no level difference with respect to the needle (in other words, the so-called "step effect" does not occur), and therefore provides an appropriate solution to the resistence's problems of the tissues at the time of inserting the needle under working conditions, avoiding the occurrence of the so-called "capping off', respectively the occurrence in which the protective sheath of the sensor is quickly ruined, thus also solving the problems of cleaning.

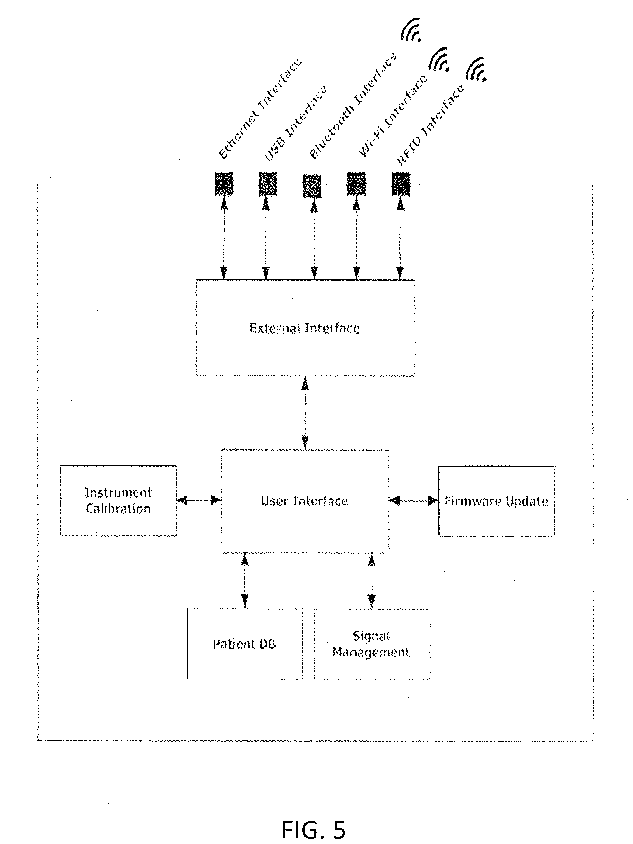

In a preferred variation, as represented in Figure 5, the apparatus is provided with software means that allows to store all the parameters, pre and post electroporation, detected by said sensors so as to vary the electrical parameters to be used on patients, manually respectively automatically, on the basis of the obtained parameters.

The parameters detected by each device of the apparatus are stored in a central database with possibility to build an intranet system with further machines capable of self-learning on the basis of the received information, suchj as in theneural network type systems.

The use of the apparatus in the theranostics field allows to define the chemical and physical characteristics of the diseases to be treated and to accordingly modify the equipment settings and the treatment parameters, such as the generated electric pulse characteristics.

In other words, the apparatus is capable of providing a sequence of actions the diagnostic phase, the therapeutic and monitoring of diseases, the personalizing of the above actions, on the basis of the kind of disease and the chemical and physical characteristics of the same.

The invention provides a more versatile instrumental solution than those currently used according to the prior art, allowing to perform the most effective procedures, in a more safely manner and with less morbility for the patient and the operator, that are also applicable in a larger range of clinical conditions, if compared with existing technologies.

Said apparatus, by means of the data collection relating to each single tumor lesion, made through its sensors, allows the phenotyping of a neoplastic mass and, consequently, a targeted treatment, aimed at maximizing the effectiveness of the therapy session.

The therapeutic and diagnostic value of the equipment allows a modulation of the therapy based on the characteristics of the tumor lesion and the patient's physiological condition.

All this is coherent with the principle of the customization of tumoral therapy (target therapy), although with the difference that the target of the therapy is not the single principal genetic alteration of the examined tumor, but its chemical, physical and biological phenotype.

This flexibility of the apparatus allows the use of the method on a very broad spectrum of diseases, even exceeding those exclusively of oncological nature.

This method also allows amplifying the effectiveness of drugs currently in use (biological and organic chemotherapy) and also allows the recovery of drugs with a too narrow therapeutic index which, for that reason, are not currently admitted in the memorandum of oncology.

The treatment is associated with reduced side effects (reduced morbility and mortality for patients) with consequent reduction of the biological and economic costs forthe health system.

### Method for using the apparatus according to the invention

For the sole purpose of further facilitating the intelligibility of the present invention, it is provided, in the following, an exemplary descriptionof a method for using the apparatus according to the invention.

Said process begins with the acquisition of information through the sensors of which the apparatus is equipped, thanks to which the more suitable type of pulses it is chosen according to the charachteristics of the situation; it is then performed the application on the patient and the re-acquisition of the sensor data to assess the occurred changes. The collected data will be stored in a dedicated memory.

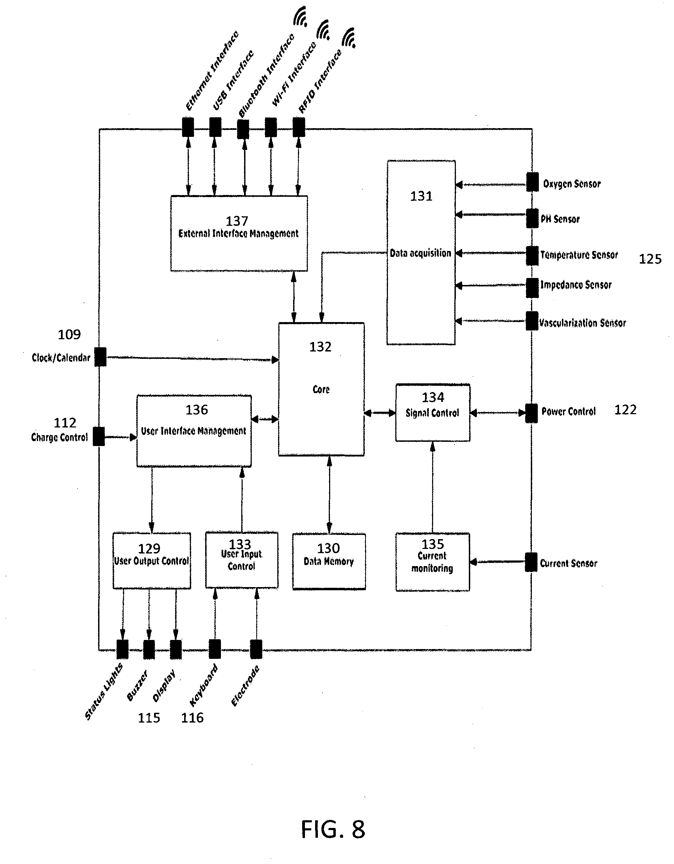

In particular, said method includes the following steps, involving the components of the apparatus, as shown in Figure 8:
1. Through the display 115, run by an User Control Output module 129, the user is invited to open a work session and to include identifying information (identity of the patient, his pathology, etc.) by using the Keyboard 116, or reading a card through the RFID interface or through a PC. All the data are stored in the Data Memory 130, temporally identifying them thanks to information received from the Clock / Calendar 109.
2. Data are collected from the sensors 125, by means of placing the same on the parts of the body of the subject to be treated. The data acquisition module 131 is responsible for collecting the response data of the sensors (Oxygen, pH, temperature, Impedance and vascularization), and processing the measurement in the Core 132 and store it in the data memory 130.
3. The Core 132 compares the collected measurements with a content data table stored in a database of the Data Memory 130, previously preloaded by one of the interfacing means (Ethernet, USB, Bluetooth and Wi-Fi by the External Interface Management module), according to which the user is offered the most appropriate pulses sequence of on the basis of the detected situation.
4. Through the display 115 and keyboard 116, the user can accept the suggested sequence of pulses or edit the parameters of the same according to their own experience.
5. The application itself is performed on the patient: the user applies the electrode on the part to be treated and press a button that the User Input Control Module 133 detects via the input 124. The Core Electrode 132 activates the Signal Control Module 134 which generates the sequence of pulses selected through the Power Control 122 output; the Current Monitoring module monitors the generated current by informing the Signal Control 135, and then the core 132, in the event that it exceeds the allowed level; if that happens, the user is immediately informed.
6. The Core 132 provides to store the treatment data in the sessions database, which resides in the data memory 130.
7. The user is prompted to use the sensors to acquire again the measures after treatment; also said data are stored in the session database residing in the Data Memory 130.
8. The user is given the option to store session data even on a personal card through RFID Interface or in a database on the PC through interface means.
9. The session is closed.

During the charging of the batteries, the User Interface Management module 136 monitors the charge status through the Charge Controller 112 and informs the user through the Status Light 113.

The User Interface Management module 136 also communicates to the Core 132 the presence of a connection with respect to a power supply networkduring recharging, so that the possibility of instrument's use is inhibited for patient safety reasons.

By means of interfacing with an external Ethernet, USB, Bluetooth and Wi-Fi by means of the External Interface Management module 137, not only it is possibile to update the user data database for choosing the most appropriate sequence of pulses, but also to transfer session data to a personal computer, in order to create a more extensive database useful both for the data storage and for a statistical treatment of the same.

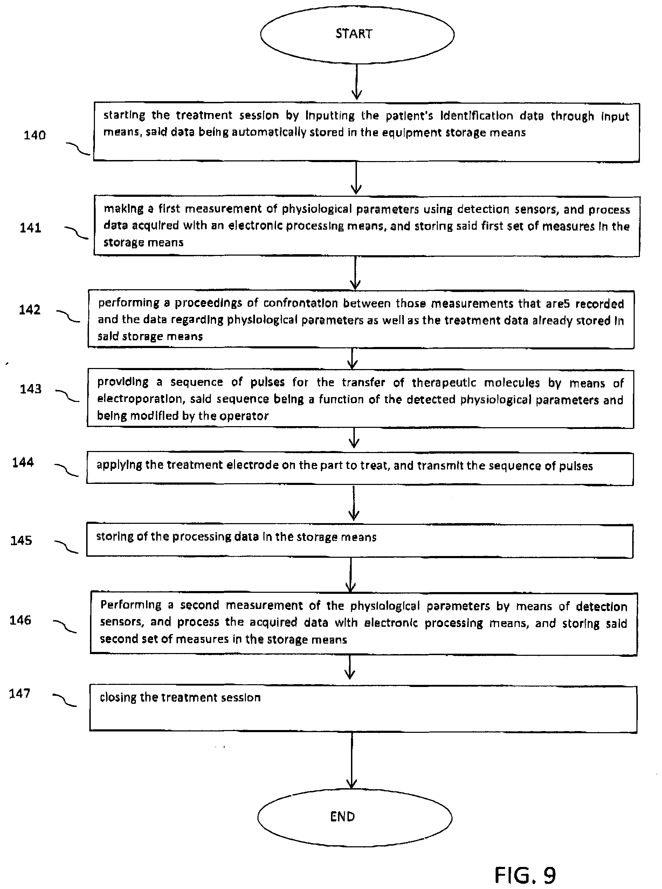

Particularly, said process comprises the following steps consisting in (Figure 9):
- Starting the treatment session by inputting the patient's identification data through input means, said data being automatically stored in the equipment storage means 140;
- Making a first measurement of physiological parameters using detection sensors, and process data acquired with an electronic processing means, and storing said first set of measures in the storage means 141;
- Performing a proceedings of confrontation between those measurements that are recorded and the data regarding physiological parameters as well as the treatment data already stored in said storage means, 142;
- Providing a sequence of pulses for the transfer of therapeutic molecules by means of electroporation, said sequence being a function of the detected physiological parameters and being modified by the operator 143;
- Applying the treatment electrode on the part to treat, and transmit the sequence of pulses 144;
- Storing of the processing data in the storage means145;
- Performing a second measurement of the physiological parameters by means of detection sensors, and process the acquired data with electronic processing means, and storing said second set of measures in the storage means 146;
- Closing the treatment session 147.

### Therapeutic applications of the apparatus according to the present invention

In the same perspective of facilitating the intelligibility of the present invention, it is it is provided, in the following, an exemplary description of the different, possible therapeutic applications of the apparatus according to the invention.

First of all, it is specified that this equipment and its Protocols are designed essentially for the loco-regional treatment of solid tumors (in veterinary medicine). By way of example, the histotypes that are currently more treated are:
- Soft tissue sarcomas
- Mastocytoma of the soft tissues
- Squamous cell carcinomas and actinic not
- Melanomas of the oral cavity
- Other oral cancer (sarcomas, carcinomas, tumors odontogenici)
- Carcinoma of skin adnexal structures
- Adenomas and carcinomas perianal and anal sac
- Lymphoma localized (skin, oral, nasal)
- Tumors of the sheaths of peripheral nerves (eg. Schwannoma)
- Benign and malignant tumors of vascular origin (eg. Haemangiopericytoma)
- Mammary tumors
- Venereal tumors.

Said apparatus is currently under extension for the use in the treatment of visceral tumors and pararectal, such as:
- thymomas
- Prostatic Carcinoma
- Tumors of the anal cavity.

Particularly, in this last type of cancer, an electrode needle with glass ceramic shielding has been employed.

The use protocol only comprises for systemic bleomycin thymomas and systemic cisplatin bleomycin intratumoral for the other histological types for the treatment of benign and viral lesions: transmissible venereal tumors, fibromatosis, equine sarcoid. The equipment and the related protocols are also designed for the treatment of viral benigne solid tumorsor low malignancy tumors, such as fibromatosis and equines sarcomas. Finally, these protocols will be suitable for the application on humans in benign or low malignancy type lesions such as:
- Viral warts
- Keloids scar
- Pediatric Fibromatosis
- Benign tumors or skin's local malignancy
- Chronic autoimmune inflammatory lesions (Lichen, annular granuloma, etc.)
- Medical Applications aesthetics and cosmetics
- Gene therapy plasmids, oligoantisense, proteins etc. (Example: retinal diseases of genetic basis).

In the case of electroporation treatments made in support of ECT and radiotherapy, performed on patient under local anesthesia and / or sedation with injective anesthetics, it can be carried out according to 3 operating modes:
1. Adjuvant Mode (sterilization of lateral and deep margins after surgical resection). In case of a superficial lesion, it is sufficient a local injection of chemotherapeutic drug (bleomicin or cisplatin, drugs of choice). After 5 minutes, so as to obtain a distribution as homogeneous as possible, there the elettroporante treatment is provided by applying trains of eight pulses of this type:
   - Positive pulse 50 µs
   - Pause 10 µs
   - Negative pulse 50 µs
   - Pause 300 µs
   and with amplitude 1300 V / cm (800 V / cm in the case of ulcerated lesions or lesions localized to mucosal level). In the case of deep lesions it is associated with local injection of cisplatin systemic administration of bleomycin.
2. Primary mode (direct attack to elettrochemiosensibile neoplasia): in case of a plaque or very superficial lesion (eg. Actinic carcinoma in eyelid localization), a systemic administration of bleomycin is carried out and followed, after 5 minutes, by the application of biphasic pulse trains described in step 1. If the lesion is volumetrically important, it may be associated with intratumoral administration of cisplatin or other antitumor drug.
3. Neoadjuvant mode, a combination of systemic and intratumoral chemotherapy as previously described is administered, followed by an electroporation in order to reduce the tumor volume and allow surgical resection.

In the light of the foregoing, it appears with evidence that the apparatus according to the invention, achieves the above indicated objets.

The subject-matter of the invention is susceptible of numerous modifications and variations, without departing from the inventive concept as expressed in the accompanying claims.

All the above-described details may furthermore be replaced with other technically equivalent elements, and the materials may be different according to the single needs, without departing from the scope of protection of the present invention. Although the present invention has been described with particular reference to the accompanying drawings, the reference numbers used in the description and in the claims are used to improve the intelligibility of the invention and do not represent any limitation to the scope of protection claimed.

It is below provided, only for clarity's sakeness, a legend of the numerical references used:
100 - main body provided with processing means and storage means;
101 - means for input of the device, in such as keyboard and / or i touch screen;
102 - probe with electrode for the treatment of lesions;
103 - probe with at least one invasive sensor, wherein the sensor penetrates inside the part for which you want to acquire a measurement;
104 - probe with at least a non-invasive type sensor, wherein the sensor is supported by the part to be measured without penetrating;
105 - probes connectors equipped with electrode and probes equipped with sensor;
106 - hardware and software visualization means, for example in the shape of a tablet computer or smartphone, capable of wired or wireless connection;
107 - connection means with respect to Internet network and / or capable for the data transmission;
108 - microcontroller unit, suitable to be used for the computer equipment management, being equipped with a remotely upgradeable firmware via an external personal computers;
109 - clock / calendar units, designed to provide information regarding date and time to the microcontroller unit for the temporal location of the archived data;
110 - external power supply for battery charging (charger);
111 - battery;
112 - charge control circuit;
113 - light for the indication of battery charge's state;
114 - buzzer means to emit sound signals;
115 - display, adapted to provide information to the user;
116 - integrated keypad, capable of receiving information from the user for the controlling and management of the equipment;
117 - communication means suitable for the data transmission and reception through an ethernet interface for LAN connections;
118 - USB interface for PC connection and pendrive;
119 - Bluetooth interface for PC connections and cell phones;
120 - Wi-Fi interface for PC connections and mobile phones;
121 - RFID interface for reading and writing data to card equipped with transponders;
122 - power control unit capable for transforming the signals generated from the microcontroller into power pulses;
123 - current sensor unit for measuring the current output, by informing the microcontroller unit that accordingly provides limiting it to a predetermined value and to verify that the current energy supply. In both cases, the user is informed regarding possibile anomalies related to excessive or lack of current output;
124 - electrode for delivering the pulses for electroporation, said pulses being generated from the microcontroller and characterized by user-settable parameters that control amplitude, number and duration;
125 - sensors connected to the equipment for measuring the temperature, pH, impedance, oxygen concentration, vascularization and / or other parameters useful for the diagnosis and monitoring of diseases, particularly tumor cells.

## Claims

1. Theranostic apparatus for therapeutic transfer of molecules by means of electroporation comprising:
- a main body (100) provided with electronic processor means and data storage means,
- input means (101), such as a keyboard and/or touch screen,
- at least one treatment probe (102) provided with an electrode, said electrode being configured to deliver electrical pulse sequences by means of electroporation,
- a plurality of probe connectors (105), for said at least one treatment probe (102),hardware and software means (106) configured for displaying data, respectively for providing sound signals,
- connection means (107) with respect to an internet network for data transmission, by means of the same,
- a power supply circuit, including batteries and a connection interface to an external power source, and
- a micro-controller unit (108), operationally connected to said electronic processor means and data storage means,
- at least one detection probe (103, 104), configured for measuring a plurality of physiological parameters,
wherein said probe comprises one or more sensors (125, 126), configured for measuring a plurality of physiological parameters, having prognostic value, of a subject undergoing the treatment,
**and** wherein said micro controller unit (108) is configured for the acquisition and processing of said physiological parameters having prognostic value, measured by at least one detecting probe (103, 104), and for the subsequent generation and control of an electroporation pulses sequence, on the basis of said processed physiological parameters having prognostic value,
**characterized in that** said microcontroller unit (108) is further configured for the determination and control of the electroporation pulses sequence for the loco-regional treatment of disorders comprised in the group of solid neoplasm, visceral and pararectal tumors, benign or low malignancy solid viral growths, benign or low malignancy lesions.

2. Apparatus according to claim **1 characterized in that** said at least one treatment probe (102) is provided with an electrode comprising two or four medical grade steel needles, partially covered by insulating glass ceramic, the ends of said needles being made of or covered by electrical conductive material.

3. Apparatus according to claim **1 characterized in that** said at least one treatment probe (102) is provided with an electrode comprising two medical grade steel plates, partially covered by insulating glass ceramic, the ends of said plates being realized or covered by electrical conductive material.

4. Apparatus according to one or more of the preceding claims **characterized in that** said at least one treatment probe (102) is configured to provide electrical pulse sequences with an energy release according to incremental step mode.

5. Apparatus according to claim 4 **characterized in that** said apparatus is configured to provide at least three amperage value measurements within the first 50 µs of the pulse electroporation sequence.

6. Apparatus according to claim 5 **characterized in that** said micro-controller unit (108) is configured for determining the automatic transmission interruption of said electroporation pulses when the amperage value is equal or higher than five ampere.

7. Apparatus according to claim 1 **characterized in that** said one or more sensors (125, 126) are configured to measure, individually or in combination, one or more parameters relating to temperature, pH, impedance, oxygen concentration, vascularization, respectively for the determination of the pathologies phenotyping.

8. Apparatus according to claim 7 **characterized in that** said one or more sensors (125, 126) are invasive, configured for measuring physiological parameters by means of insertion.

9. Apparatus according to claim 7 **characterized in that** said one or more sensors (125, 126) are non-invasive, configured for measuring physiological parameters transcutaneously.

10. Apparatus according to one or more of the preceding claims **characterized in that** said apparatus is configured for automatically recognizing the type of probes (102, 103, 104) and relative sensors (125, 126) connected to the same, and for the acquisition of the relating working information.

11. Apparatus according to one or more of the preceding claims **characterized in that** said at least one treatment probe (102), respectively said at least one detection probe (103, 104), includes an RFID module for the tracking and storing the number of uses within a predetermined time unit.

12. Apparatus according to one or more of the preceding claims **characterized in that** said storage means of said main body (100),operatively connected to the said microcontroller unit (108), comprise a database for the storage of a plurality of physiological parameters, detected by means of said sensors (125, 126) before, respectively after, the transmission of the electroporation pulses sequence, upon instruction from said microcontroller unit (108) and **in that** said microcontroller unit (108) is implemented with software means configured for the elaboration of said parameters and for the subsequent automatic regulation of the electroporation sequence of pulses to be transmitted, on the basis of the same.

## Patentansprüche

1. Theranostische Vorrichtung zum therapeutischen Transfer von Molekülen mittels Elektroporation, umfassend:
- einen Hauptkörper (100), der mit elektronischen Prozessormitteln und Datenspeichermitteln versehen ist,
- Eingabemittel (101), wie eine Tastatur und/oder einen Touchscreen,
- mindestens eine Behandlungssonde (102), die mit einer Elektrode versehen ist, wobei die Elektrode dazu eingerichtet ist, elektrische Impulsfolgen mittels Elektroporation zu übermitteln,
- eine Vielzahl von Sondensteckvorrichtungen (105) für die mindestens eine Behandlungssonde (102), Hardware- und Softwaremittel (106), die zur Datenanzeige beziehungsweise zum Bereitstellen von Tonsignalen eingerichtet sind,
- Anschlussmittel (107) in Bezug auf ein Internet-Netz für die Datenübertragung mithilfe desselben,
- eine Stromversorgungsschaltung, umfassend Batterien und eine Anschlussschnittstelle für eine externe Stromquelle, und
- eine Mikrocontrollereinheit (108), die betrieblich mit den elektronischen Prozessormitteln und den Datenspeichermitteln verbunden ist,
- mindestens eine Detektionssonde (103, 104), die zum Messen einer Vielzahl physiologischer Parameter eingerichtet ist,
wobei die Sonde einen oder mehr Sensoren (125, 126) umfasst, die zum Messen einer Vielzahl physiologischer Parameter mit prognostischem Wert einer der Behandlung unterzogenen Person eingerichtet sind,
und wobei die Mikrocontrollereinheit (108) zum Erfassen und Verarbeiten der physiologischen Parameter mit prognostischem Wert, die mit mindestens einer Detektionssonde (103, 104) gemessen werden, und zum anschließenden Erzeugen und Steuern einer Elektroporationsimpulsfolge auf der Grundlage der verarbeiteten physiologischen Parameter mit prognostischem Wert eingerichtet ist,
**dadurch gekennzeichnet, dass** die Mikrocontrollereinheit (108) ferner zum Bestimmen und Steuern der Elektroporationsimpulsfolge für die lokoregionäre Behandlung von Erkrankungen eingerichtet ist, die in der Gruppe aus solidem Neoplasma, viszeralen und pararektalen Tumoren, benignen oder niedrigmalignen soliden viralen Wucherungen und benignen oder niedrigmalignen Läsionen enthalten sind.

2. Vorrichtung nach Anspruch 1, **dadurch gekennzeichnet, dass** die mindestens eine Behandlungssonde (102) mit einer Elektrode versehen ist, die zwei oder vier Stahlnadeln medizinischer Güte umfasst, die teilweise mit isolierender Glaskeramik überzogen sind, wobei die Enden der Nadeln aus einem elektrisch leitenden Material bestehen oder damit überzogen sind.

3. Vorrichtung nach Anspruch 1, **dadurch gekennzeichnet, dass** die mindestens eine Behandlungssonde (102) mit einer Elektrode versehen ist, die zwei Stahlplatten medizinischer Güte umfasst, die teilweise mit isolierender Glaskeramik überzogen sind, wobei die Enden der Platten mit einem elektrisch leitenden Material ausgeführt oder überzogen sind.

4. Vorrichtung nach einem oder mehreren der vorhergehenden Ansprüche, **dadurch gekennzeichnet, dass** die mindestens eine Behandlungssonde (102) dazu eingerichtet ist, elektrische Impulsfolgen mit einer Energieabgabe gemäß einem inkrementellen Schrittmodus bereitzustellen.

5. Vorrichtung nach Anspruch 4, **dadurch gekennzeichnet, dass** die Vorrichtung dazu eingerichtet ist, mindestens drei Messungen des Stromstärkewerts innerhalb der ersten 50 µs der Elektroporationsimpulsfolge bereitzustellen.

6. Vorrichtung nach Anspruch 5, **dadurch gekennzeichnet, dass** die Mikrocontrollereinheit (108) dazu eingerichtet ist, die automatische Unterbrechung der Übertragung der Elektroporationsimpulse zu bedingen, wenn der Stromstärkewert gleich oder höher als fünf Ampere ist.

7. Vorrichtung nach Anspruch 1, **dadurch gekennzeichnet, dass** der eine oder die mehreren Sensoren (125, 126) dazu eingerichtet sind, einzeln oder in Kombination einen oder mehr Parameter, die sich auf die Temperatur, den pH-Wert, die Impedanz, die Sauerstoffkonzentration beziehungsweise die Vaskularisation beziehen, zum Bestimmen der Phänotypisierung der Pathologien zu messen.

8. Vorrichtung nach Anspruch 7, **dadurch gekennzeichnet, dass** der eine oder die mehreren Sensoren (125, 126) invasiv sind, dazu eingerichtet, physiologische Parameter durch Einführen zu messen.

9. Vorrichtung nach Anspruch 7, **dadurch gekennzeichnet, dass** der eine oder die mehreren Sensoren (125, 126) nicht-invasiv sind, dazu eingerichtet, physiologische Parameter transkutan zu messen.

10. Vorrichtung nach einem oder mehreren der vorhergehenden Ansprüche, **dadurch gekennzeichnet, dass** die Vorrichtung dazu eingerichtet ist, die Art der Sonden (102, 103, 104) und der jeweiligen daran angeschlossenen Sensoren (125, 126) automatisch zu erkennen und die entsprechenden Arbeitsinformationen zu erfassen.

11. Vorrichtung nach einem oder mehreren der vorhergehenden Ansprüche, **dadurch gekennzeichnet, dass** die mindestens eine Behandlungssonde (102) beziehungsweise die mindestens eine Detektionssonde (103, 104) ein RFID-Modul zum Verfolgen und Speichern der Anzahl der Verwendungen innerhalb einer vorbestimmten Zeiteinheit umfasst.

12. Vorrichtung nach einem oder mehreren der vorhergehenden Ansprüche, **dadurch gekennzeichnet, dass** die Datenspeichermittel des Hauptkörpers (100), die mit der Mikrocontrollereinheit (108) betrieblich verbunden sind, eine Datenbank zum Speichern einer Vielzahl physiologischer Parameter, die mithilfe der Sensoren (125, 126) vor beziehungsweise nach der Übertragung der Elektroporationsimpulsfolge detektiert werden, auf Anweisung der Mikrocontrollereinheit (108) umfassen, und dadurch, dass die Mikrocontrollereinheit (108) mit Softwaremitteln implementiert ist, die für die Ausarbeitung dieser Parameter und für die anschließende automatische Regelung der zu übertragenden Elektroporationsimpulsfolge auf deren Grundlage eingerichtet sind.

## Revendications

1. Appareil théranostique pour le transfert thérapeutique de molécules par une électroporation comprenant :
- un corps principal (100) pourvu de moyens de processeur électronique et de moyens de stockage de données,
- des moyens de saisie (101), tels qu'un clavier et/ou un écran tactile,
- au moins une sonde de traitement (102) pourvue d'une électrode, ladite électrode étant conçue pour délivrer des séquences d'impulsions électriques par électroporation,
- une pluralité de connecteurs de sonde (105), pour ladite au moins une sonde de traitement (102), des moyens matériels et logiciels (106) conçus pour afficher des données, respectivement pour fournir des signaux sonores,
- des moyens de connexion (107) par rapport à un réseau Internet pour la transmission de données, au moyen de ceux-ci,
- un circuit d'alimentation électrique, comprenant des batteries et une interface de connexion à une source d'alimentation externe et
- une unité de microcontrôleur (108), connectée fonctionnellement auxdits moyens de processeur électronique et auxdits moyens de stockage de données,
- au moins une sonde de détection (103, 104), conçue pour mesurer une pluralité de paramètres physiologiques,
ladite sonde comprenant un ou plusieurs capteurs (125, 126), conçus pour mesurer une pluralité de paramètres physiologiques, présentant une valeur de pronostic, d'un sujet subissant le traitement
et ladite unité de microcontrôleur (108) étant conçue pour l'acquisition et le traitement desdits paramètres physiologiques présentant une valeur de pronostic, mesurés par au moins une sonde de détection (103, 104), et pour la génération et la commande ultérieures d'une séquence d'impulsions d'électroporation, sur la base desdits paramètres physiologiques traités présentant une valeur de pronostic
**caractérisé en ce que** ladite unité de microcontrôleur (108) est en outre conçue pour la détermination et la commande de la séquence d'impulsions d'électroporation pour le traitement loco-régional de troubles compris dans le groupe constitué par un néoplasme solide, les tumeurs viscérales et pararectales, les croissances virales solides bénignes ou de faible malignité, les lésions bénignes ou de faible malignité.

2. Appareil selon la revendication 1, **caractérisé en ce que** ladite au moins une sonde de traitement (102) est pourvue d'une électrode comprenant deux ou quatre aiguilles en acier de qualité médicale, partiellement recouvertes par une vitrocéramique isolante, les extrémités desdites aiguilles étant constituées ou recouvertes par un matériau conducteur électrique.

3. Appareil selon la revendication 1, **caractérisé en ce que** ladite au moins une sonde de traitement (102) est pourvue d'une électrode comprenant deux plaques en acier de qualité médicale, partiellement recouvertes par une vitrocéramique isolante, les extrémités desdites plaques étant réalisées en un matériau conducteur électrique ou recouvertes par celui-ci.

4. Appareil selon l'une ou plusieurs des revendications précédentes, **caractérisé en ce que** ladite au moins une sonde de traitement (102) est conçue pour fournir des séquences d'impulsions électriques avec une libération d'énergie selon un mode par pas incrémentiels.

5. Appareil selon la revendication 4, **caractérisé en ce que** ledit appareil est conçu pour fournir au moins trois mesures de valeur d'ampérage dans les premières 50 µs de la séquence d'impulsions d'électroporation.

6. Appareil selon la revendication 5, **caractérisé en ce que** ladite unité de microcontrôleur (108) est conçue pour déterminer l'interruption de transmission automatique desdites impulsions d'électroporation lorsque la valeur d'ampérage est égale ou supérieure à cinq ampères.

7. Appareil selon la revendication 1, **caractérisé en ce que** ledit un ou lesdits plusieurs capteurs (125, 126) sont conçus pour mesurer, individuellement ou en combinaison, un ou plusieurs paramètres relatifs à la température, au pH, à l'impédance, à la concentration en oxygène, à la vascularisation, respectivement pour déterminer le phénotypage de pathologies.

8. Appareil selon la revendication 7, **caractérisé en ce que** ledit un ou lesdits plusieurs capteurs (125, 126) sont invasifs, conçus pour mesurer des paramètres physiologiques par insertion.

9. Appareil selon la revendication 7, **caractérisé en ce que** ledit un ou lesdits plusieurs capteurs (125, 126) sont non invasifs, conçus pour mesurer des paramètres physiologiques par voie transcutanée.

10. Appareil selon l'une ou plusieurs des revendications précédentes, **caractérisé en ce que** ledit appareil est conçu pour reconnaître automatiquement le type des sondes (102, 103, 104) et des capteurs relatifs (125, 126) connectés à ceux-ci et pour acquérir les informations de travail associées.

11. Appareil selon l'une ou plusieurs des revendications précédentes, **caractérisé en ce que** ladite au moins une sonde de traitement (102), respectivement ladite au moins une sonde de détection (103, 104), comprend un module RFID pour le suivi et le stockage du nombre d'utilisations au sein d'une unité de temps prédéterminée.

12. Appareil selon l'une ou plusieurs des revendications précédentes, **caractérisé en ce que** lesdits moyens de stockage dudit corps principal (100), connectés fonctionnellement à ladite unité de microcontrôleur (108), comprennent une base de données pour le stockage d'une pluralité de paramètres physiologiques, détectés au moyen desdits capteurs (125, 126) avant, respectivement après, la transmission de la séquence d'impulsions d'électroporation, suite à une instruction provenant de ladite unité de microcontrôleur (108) et **en ce que** ladite unité de microcontrôleur (108) est mise en oeuvre avec des moyens logiciels conçus pour l'élaboration desdits paramètres et pour la régulation automatique ultérieure de la séquence d'impulsions d'électroporation à transmettre, sur la base de ceux-ci.
